# EUROPÄISCHE PATENTSCHRIFT

(11) **EP 2 531 171 B1**
(45) Veröffentlichungstag und Bekanntmachung des Hinweises auf die Patenterteilung: **06.07.2016**
(21) Anmeldenummer: 11703593.1
(22) Anmeldetag: 02.02.2011
(51) Int. Cl.: A61K 8/891, A61Q 1/06, A61Q 1/08, A61Q 1/10, A61K 8/86, A61K 8/02, A61K 8/81

(54) **KOSMETISCHE ZUBEREITUNG UND IHRE VERWENDUNG**
COSMETIC FORMULATION AND USE THEREOF
PRÉPARATION COSMÉTIQUE ET UTILISATION DE CELLE-CI

(30) Priorität: 02.02.2010 DE 202010001688 U
(43) Veröffentlichungstag der Anmeldung: 12.12.2012
(73) Patentinhaber: Schwan-STABILO Cosmetics GmbH & Co. KG, 90562 Heroldsberg (DE)
(72) Erfinder: HELL, Sigrid, 91166 Georgensgmünd (DE); LEBOK, Simona, 90482 Nürnberg (DE); STRAUBINGER, Marika, 91096 Möhrendorf (DE)
(74) Vertreter: Leissler-Gerstl, Gabriele
(86) Internationale Anmeldenummer: PCT/EP2011/000474
(87) Internationale Veröffentlichungsnummer: WO 2011/095331

(56) Entgegenhaltungen:
- DE-A1-102007 038 936
- US-A1- 2006 134 035
- US-A1- 2006 280 763
- US-A1- 2008 171 006
- US-A1- 2009 092 567

## Beschreibung

Die Erfindung betrifft eine kosmetische Zubereitung sowie deren Verwendung.

Kosmetikprodukte sind aus der heutigen Zeit nicht mehr wegzudenken. Je schnelllebiger die Zeit wird, desto höhere Anforderungen werden auch an die jeweiligen Kosmetika gestellt. Während man früher das Schminken und die Pflege der Haut und des Körpers noch als Genuss erlebt hat, muss das perfekte Aussehen heute schon mit möglichst nur einem Handgriff zu erzielen sein. Eine Folge davon sind Produkte mit einer Vielzahl an Inhaltsstoffen, die gleichzeitig und bevorzugt schon bei einmaliger Anwendung gegen viele Hautdefekte helfen sollen. Während dekorative Effekte der Kosmetika dabei sofort visualisiert werden können, wird die Langzeitwirkung der Produkte durch aktive Inhaltsstoffe erzielt.

Damit die vorteilhaften Effekte der kosmetischen Zubereitungen auch dauerhaft erzielt werden können, müssen die Kosmetikprodukte über eine ausgezeichnete Haftung auf dem Applikationsort, also zum Beispiel der Haut und ihren Hautanhangsgebilden, den Haaren, den Schleimhäuten und Semischleimhäuten, aufweisen. Ebenso ist es wichtig, dass die Produkte wasserfest sind und sich nicht auf Gegenstände oder Hautpartien sowie Textilien übertragen, mit denen sie in Berührung kommen, was landläufig als "no transfer Effekt" bezeichnet wird.

Handelsübliche Kosmetikprodukte, die sowohl wasserresistent sind als auch über einen no transfer Effekt verfügen, werden zumeist dadurch erhalten, dass man üblichen Wachsmischungen flüchtige Silikonverbindungen zusetzt, die die Wachszubereitung beim Auftrag geschmeidig halten, und nach ihrem Abdampfen den Wachsfilm auf dem Applikationsareal hinterlassen. Nachteilig hieran ist, dass der resultierende "abgetrocknete" Wachsfilm durch den Verlust des legierenden, silikonhaltigen Solubilisierungsmittels oft brüchig oder rissig ist und eine matte, stumpfe Oberfläche aufweist, die am Applikationsort spannt oder brennt und während der Tragedauer ein unangenehmes Gefühl hinterlässt. Um diesen Nachteil zu überwinden wird diesen rein wachsbasierten Zubereitungen ein Anteil an lipophilen Ölen beigefügt, die das Wachsgefüge geschmeidig halten sollen. Nachteilig hieran ist aber, dass gerade diese auf der Haut verbleibenden Öle durch Kapillareffekte bevorzugt vom Applikationsort abwandern, was die Haftung der Zubereitung am Applikationsort deutlich senkt und zu Sekundäreffekten, wie dem Auslaufen der Zubereitung auf umgebende Haut- oder Haarpartien, dem sogenannten "Ausbluten", führt, was insbesondere bei farbigen Zubereitungen intolerabel ist.

Als Alternative zu silikonhaltigen Wachskosmetika werden wasserbasierte Kosmetikprodukte auf Emulsionsbasis oder Suspensionsbasis angeboten, deren Haltbarkeit auf dem Applikationsareal durch spezifische polymere Inhaltsstoffe bereitgestellt wird. Diese Polymere bilden nach dem Abdampfen des Lösungsmittels einen zumindest teilweise wasserfesten Film, der je nach verwendetem Polymer mehr oder weniger stark glänzend ist. Nachteilig an solchen wasserbasierten Systemen ist, dass diese in jedem Fall Tenside und/oder Emulgatoren erfordern, damit das wasserhaltige Produkt stabil und auf der Haut verteilbar ist und nicht von dort abperlt. Diese Tenside und/oder Emulgatoren wirken sich aber nachteilig auf die Wasserfestigkeit und mechanische Beständigkeit des gebildeten kosmetischen Films aus, denn z.B. in Berührung mit Wasser oder anderen protischen Lösungsmitteln reemulgiert oder solubilisiert der bereits gebildete Film und löst sich vom Applikationsort ab oder reißt auf. Weiter nachteilig ist, dass zur Ausbildung eines haltbaren polymeren Films große Mengen an Polymer erforderlich sind. Dies führt ebenfalls zu einem unangenehmen Spannen des Films am Applikationsort. Dem kann nur teilweise durch Zufügung kleiner Moleküle, sogenannter Weichmacher, entgegengewirkt werden, die aber oftmals unverträglich mit den restlichen Bestandteilen der Zubereitung sind.

Aufgabe der vorliegenden Erfindung ist damit, die oben beschriebenen Nachteile bekannter kosmetischer Produkte zu überwinden und eine kosmetische Zubereitung bereitzustellen, die über hervorragende Trageeigenschaften verfügt, insbesondere am Applikationsort lange haftet, von dort nicht abwandert oder sich überträgt, wasser- und wischfest ist und daneben einfach und gleichmäßig zu applizieren ist. Darüber hinaus soll die Zubereitung auch bei erhöhten bzw. sehr niedrigen Temperaturen lagerstabil sein und üblichen mechanischen Belastungen, wie sie auf gängige Kosmetikprodukte einwirken können, standhalten. Unter "lagerstabil" wird dabei verstanden, dass die erfindungsgemäße Zubereitung bei einer vierwöchigen Lagerung bei -10 °C bis 45 °C weder optisch noch sensorisch eine merkliche Veränderung erfährt.

Die genannten Aufgaben werden durch eine kosmetische Zubereitung gelöst, wie sie in Anspruch 1 definiert ist. Die Unteransprüche haben vorteilhafte Weiterbildungen zum Gegenstand.

Gegenstand der Erfindung ist eine kosmetische Zubereitung, die mindestens ein Silsesquioxanwachs der Formel (R₂R'SiO_{1/2})ₓ(R"SiO_{3/2})_{y} und mindestens einen Thermoplast mit mindestens einer von Cyclopentadien abgeleiteten Einheit enthält, wobei der Thermoplast hydriertes Polydicyclopentadien ist. In oben dargestellter Formel ist R jeweils eine Alkylgruppe mit 1 bis 8 C-Atomen, R' eine einwertige Alkylgruppe mit 9 bis 46 C-Atomen, R" eine einwertige Alkylgruppe mit 1 bis 8 C-Atomen oder eine Arylgruppe, wobei x und y Werte zwischen 0,05 und 0,95 annehmen können. Das Silsesquioxanwachs kann neben den oben genannten Gruppen auch weitere Siloxy-Einheiten aufweisen, wie zum Beispiel (R¹₃SiO_{1/2})ₐ, (R²₂SiO_{2/2})_{b}, (R³SiO_{3/2})_{c} oder (SiO_{4/2})_{d}, die auch als M-, D-, T- und Q-Einheiten bezeichnet werden und allgemein bekannt sind. Das beschriebene Silsesquioxanwachs ist in gängigen Wachs- bzw. Ölmischungen löslich oder zumindest homogen verteilbar und wirkt als Strukturgeber in der umgebenden Masse. Durch seine Struktur ist es in der Lage in der kosmetischen Zubereitung eine Matrix aufzuspannen, die als strukturgebendes Gerüst dient, in das die übrigen Komponenten eingebettet oder zumindest angelagert sind. Das Silsesquioxanwachs ist aus zwei unterschiedlichen spezifischen silikonhaltigen Einheiten aufgebaut, die dem Wachs sowohl wachsartige wie auch harzartige Eigenschaften verleihen. Aufgrund der (R"SiO_{3/2})_{y}-Einheit erstreckt sich das Molekül in der Zubereitung nicht nur in Längsrichtung, sondern weist teilquervernetzte Bereiche auf, die die Zubereitung zusätzlich stabilisieren. Mit der Länge der Reste R, R' oder R", steigt dabei der wachsartige Charakter an und das Wachs gewinnt an Lipophilie und Substantivität. Auch steigt damit der Schmelzbereich des Wachses. Durch den teillipophilen Charakter des Wachses ist es insbesondere auf Haut und Hautanhangsgebilden, sowie auch auf Schleimhäuten und Semischleimhäuten gut verteilbar und weist dazu eine hohe Adhäsion auf, so dass die kosmetische Zubereitung gut und lange am Applikationsort haftet und zudem beständig ist gegenüber Wasser. Der harzartige Charakter fördert ebenfalls die lange Haltbarkeit am Applikationsort, eben durch die Ausbildung des oben beschriebenen Matrixeffekt. Zudem werden dadurch aber auch noch die no transfer Eigenschaften der Zubereitung gefördert. Ohne an eine Theorie gebunden zu sein wird angenommen, dass durch die aufgespannte Matrix die in der kosmetischen Zubereitung ferner enthaltenen Komponenten darin so gut eingebettet sind, dass das Silsesquioxanwachs scheinbar eine Art Schutzfilm um die Komponenten legt, aus der diese nicht abwandern können. Im Ergebnis überträgt sich die Zubereitung nach Applikation nicht auf sie berührende Gegenstände, Haut- und Haarpartien oder Textilien, wie Kleidung, Servietten und dergleichen. Die erfindungsgemäße Zubereitung verbleibt dauerhaft am Applikationsort.

Die in obiger Formel zuerst genannte silikonhaltige Einheit weist drei Reste auf, von denen zwei gleich sein können. Diese Struktureinheit bildet die endständige Einheit des Grundgerüsts des Silsesquioxanwachses. R' ist ein einwertiger Kohlenwasserstoffrest mit 9 bis 46 C-Atomen im Grundgerüst. Je länger dieser Rest, desto größer ist die Lipophilie des Moleküls und damit zum Beispiel die Haftung auf Haut, Hautpartien, Schleimhäuten und dergleichen. Zudem nimmt mit zunehmender Kettenlänge des Rests R' die Verträglichkeit in gängigen lipophilen kosmetischen Rohstoffen zu, was die Stabilität der gesamten Zubereitung insbesondere bei hohen oder extrem niedrigen Temperaturen deutlich erhöht. Eine Kettenlänge von mehr als 46 C-Atomen hingegen führt jedoch zu starken Spannungen im Molekül, so dass dessen Stabilität bei mechanischer Belastung, wie sie zum Beispiel bei Applikation einer Zubereitung auf einem Hautareal auftritt, nicht standhält. Die Zubereitung wirkt brüchig und krümelt. Ein Kohlenwasserstoffrest mit mindestens 9 C-Atomen ist allerdings erforderlich um die gewünschte Lipophilie bereitzustellen. Die mit R bezeichneten Reste können jeweils gleich oder verschieden sein, sind aber bevorzugt gleich. R ist jeweils ein linearer oder verzweigter Kohlenwasserstoffrest mit 1 bis 8 Kohlenstoffen in seinem Grundgerüst. Je längerkettig diese zweiten Kohlenwasserstoffreste R, desto gespannter ist das Molekül an seinen Enden, da sich die Ketten der Reste R mit denjenigen des Restes R' teilweise überlagern bzw. sterisch beeinflussen, was trotz der chemischen Ähnlichkeit zu sterischen innermolekularen Spannungen führen kann. Zudem führen große Reste R eher zur teilweisen Blockade der durch das Silsesquioxan aufgespannten Kavitäten, die eigentlich für die übrigen Komponenten der Zubereitung zur Verfügung stehen sollen. Die Kettenlänge der Reste R sollten daher 8 C-Atome nicht überschreiten. Die in obiger Formel zuletzt genannte Einheit bildet nicht nur das lineare Grundgerüst des Silsesquioxanwachses, sondern ist ein vernetzendes Strukturelement, das dem Wachs einen harzartigen Charakter verleiht und daher die no transfer Eigenschaften der erfindungsgemäßen Zubereitung erhöht. Es umfasst eine Siloxaneinheit mit dem Rest R", der ein Arylrest oder ein einwertiger Kohlenwasserstoffrest mit 1 bis 8 C-Atomen sein kann. Auch hier ist es wichtig, dass die Molekülgröße bzw. Kettenlänge des Restes R" nicht zu groß ist, da ansonsten das netzartige Gebilde, das durch das Silsesquioxanwachs aufgespannt wird, durch die Reste R" teilweise gestört bzw. gefüllt wird und dieses daher nicht mehr als Stütze der erfindungsgemäßen kosmetischen Zubereitung dienen kann. Insbesondere bei kondensierten Ringsystemen bzw. Arylgruppen mit ausladenden sterisch abschirmenden Substituenten oder einem Alkylrest mit einer Kettenlänge von mehr als 8 C-Atomen ist die Wahrscheinlichkeit der durch sterisch bedingte repulsive Wechselwirkungen ausgelösten Spannungen so groß, dass die Gerüstmatrix aufgeweitet wird und damit nicht belastbar ist. Bevorzugte Kettenlängen des Restes R" sind 3 bis 6 C-Atome oder aber R" ist eine Phenylgruppe mit oder ohne Substituenten. Es hat sich herausgestellt, dass dann die Wechselwirkungen mit der Primäreinheit so gering sind, dass die üblichen kosmetischen Komponenten homogen und stabil in der Gerüststruktur des Silsesquioxanwachses eingebettet und dauerhaft darin stabilisiert sind.

Die eingesetzten Mengen des Silsesquioxanwachses sind im Einzelnen nicht beschränkt. Bevorzugt ist es aber, wenn die Menge 1 bis 20 Gew.-% und besonders bevorzugt 3 bis 15 Gew.-% bezogen auf das Gesamtgewicht der Zubereitung beträgt. Bei Mengen von weniger als 1 Gew.-% werden die vorteilhaften, wünschenswerten Eigenschaften der erfindungsgemäßen Zubereitung, wie die lange Haftung am Applikationsort oder die no transfer Eigenschaften, sowie der die Zubereitung stabilisierende Effekt weniger ausgeprägt erhalten. Mengen, die 20 Gew.-% bezogen auf das Gesamtgewicht der Zubereitung der Erfindung übersteigen sind zwar möglich, bewirken aber keine deutliche Steigerung der vorteilhaften Eigenschaften der erfindungsgemäßen Zubereitung mehr.

Wie bei anderen langkettigen Kohlenwasserstoffwachsen, also zum Beispiel hochschmelzenden Ozokeriten, mikrokristallinen Wachsen und Paraffinen, oder quervernetzenden Harzen wie Polyurethanpolymeren oder anderen funktionalen Acrylatpolymeren, weist das oben beschriebene Silsesquioxanwachs aber durch seine Teilkristallinität auch Eigenschaften auf, die sich weniger vorteilhaft auf die Haltbarkeit, besser gesagt auf das Gesamterscheinungsbild der applizierten Zubereitung, auswirken. So wurde gefunden, dass es allein nicht ausreichend ist einer üblichen Wachsmischung zur Verbesserung der Haltbarkeit am Applikationsareal das besagte Silsesquioxanwachs zuzufügen, denn auch dieses fördert die Brüchigkeit und Rissigkeit der umgebenen Wachsmasse, da der elastische Anteil in dem Silsesquioxanwachs geringer ist.

Es wurde nun überraschend gefunden, dass eine kosmetische Zubereitung, die das beschriebene Silsesquioxanwachs in Kombination mit mindestens einem Thermoplast mit mindestens einer von Cyclopentadien abgeleiteten Einheit enthält, wobei der Thermoplast hydriertes Polydicyclopentadien ist, über eine ausgezeichnete dauerhafte Haftung am Applikationsareal verfügt und extrem wasser- und wischfest ist. Zudem überträgt sich die Zubereitung nicht auf Gegenstände, Textilien oder Haut- und Haarpartien, mit denen sie in Berührung kommt. Die Zubereitung lässt sich leicht applizieren und bildet einen sehr feinen, homogenen Film aus, der sich nicht überträgt oder vom Applikationsort abwandert und dennoch geschmeidig und angenehm weich ist. Ebenso wird durch die erfindungsgemäße Kombination von Komponenten die Migrationsneigung vermindert und die Zubereitung hält sogar Kapillareffekten, wie sie in feinen Hautfältchen wirken, stand. Einzigartig ist, dass gerade die Kombination von Silsesquioxanwachs mit einem Thermoplast mit mindestens einer von Cyclopentadien abgeleiteten Einheit einer sonst zu Brüchigkeit neigenden, wachsartigen Zubereitung eine ausreichende Geschmeidigkeit verleiht, so dass der sich nach Applikation ausbildende Film auf der Applikationsoberfläche elastisch bleibt, so dass ein Aufreißen in Hautfältchen oder Hautunebenheiten und andere Filmdefekte unterbunden werden. Die Applikationsfläche bleibt über lange Zeit gleichmäßig bedeckt, ohne auszutrocknen oder ein unangenehmes spannendes Gefühl oder gar ein Brennen zu erzeugen.

Ein Thermoplast im Sinne der Erfindung ist ein Kunststoff, der sich in einem bestimmten Temperaturbereich einfach und ohne zerstört zu werden (also thermoplastisch) verformen lässt. Dieser Vorgang ist reversibel, was bedeutet, dass er durch Abkühlung und Wiedererwärmung bis in den geschmolzenen Zustand beliebig oft wiederholt werden kann, solange nicht durch Überhitzung die sogenannte thermische Zersetzung des Thermoplasts einsetzt. Thermoplaste sind aus gering oder nicht verzweigten, also überwiegend linearen Kohlenstoffketten aufgebaut, die nur durch schwache physikalische Bindungen miteinander verbunden sind. Thermoplaste können daneben auch teilkristalline Bereiche aufweisen. In diesen Bereichen sind die Kohlenstoffketten überwiegend parallel ausgerichtet.

Erfindungsgemäß werden Thermoplaste eingesetzt, wobei mindestens ein Thermoplast mindestens eine von Cyclopentadien abgeleitete Einheit aufweist, wobei der Thermoplast hydriertes Polydicyclopentadien ist. Die von Cyclopentadien abgeleitete Einheit kann Cyclopentadien, Dicyclopentadien, Polycyclopentadien, Polydicyclopentadien oder ein hydriertes Derivat von einer der genannten Einheiten sein. Der Thermoplast kann aus nur einer dieser Einheiten oder einer Mischung von Einheiten bestehen oder auch aus Cyclopentadieneinheiten und anderen Monomeren aufgebaut sein. So kann der Thermoplast ein Homopolymer, Copolymer, Terpolymer, Crosspolymer oder ähnliches sein.

Ohne an die Theorie gebunden zu sein wird vermutet, dass sich die von Cyclopentadien ableitende Einheit besonders gut in die von dem erfindungsgemäßen Silsesquioxanwachs aufgespannte Matrix einbettet bzw. als wenig sterisch anspruchsvolles Molekül kaum repulsive Wechselwirkungen im Matrixgefüge erzeugt. Es dient gleichermaßen als Abstandshalter wie auch als Füllstoff in den Kavitäten des Wachsgerüsts. Scheinbar wirken beide Rohstoffe aufgrund ihrer Struktur so optimal zusammen, dass durch den Thermoplast eine gewisse Elastizität in den Gesamtverbund der Zubereitung eingetragen wird, indem die von Cyclopentadien abgeleitete Einheit, das starre Alkylsiloxangerüst partiell aufweitet und damit gefügig und anschmiegsam macht. Da die erfindungsgemäßen Thermoplaste aufgrund ihrer Lipophilie, die insbesondere auch durch das von Cyclopentadien abgeleitete Molekül eingetragen wird, ebenfalls die Haftung auf Haut, Hautanhangsgebilden, Schleimhäuten und dergleichen, erhöhen, wirken beide erfindungsgemäßen Komponenten in Bezug auf die Haltbarkeit am Applikationsort synergistisch zusammen, was der erfindungsgemäßen Zubereitung eine überragend lange Haftung sowie einen ausgesprochen guten no transfer Effekt verleiht.

Die Thermoplaste neben mindestens einem Thermoplasten, welcher eine von Cyclopentadien abgeleitete Einheit aufweist, wobei der Thermoplast Polydicyclopentadien ist, sind im Einzelnen nicht beschränkt. Sie müssen aber mindestens eine von Cyclopentadien abgeleitete Einheit aufweisen und sollten für kosmetische Anwendungen geeignet sein. Je größer der Anteil an von Cyclopentadien abgeleiteten Einheiten, desto ausgeprägter sind die positivierenden Eigenschaften auf die Gesamtzubereitung, die das erfindungsgemäße Silsesquioxanwachs enthält.

Der erfindungsgmäße Thermoplast, wobei der Thermoplast hydriertes Polydicyclopentadien ist, oder die Mischung aus Thermoplasten, wobei mindestens ein Thermoplast hydriertes Polydicyclopentadien ist, kann in beliebigen Mengen eingesetzt werden. Vorteilhaft ist es aber, wenn die Menge an Thermoplast 2 bis 20 Gew.-%, bevorzugt 5 bis 12 Gew.-%, bezogen auf das Gesamtgewicht der Zusammensetzung beträgt. Bei einem Gehalt von weniger als 2 Gew.-% in Bezug auf die Gesamtzusammensetzung kann der die Elastizität und Haftung fördernde Effekt in der Zubereitung nicht in gesteigertem Maße erzielt werden. Bei einem Gehalt ab 20 Gew.-% bezogen auf das Gesamtgewicht der Zubereitung können darüber hinaus keine weiteren Verbesserungen in Bezug auf die Haftung am Applikationsort erzielt werden.

Als besonders gut für die erfindungsgemäße kosmetische Zubereitung hat sich die Kombination des wie oben beschriebenen Thermoplasts mit einem Silsesquioxanwachs gemäß oben genannter Formel erwiesen, wenn in der Formel R jeweils eine Methylgruppe, R' eine Alkylgruppe mit 30 bis 46 C-Atomen und R" eine Alkylgruppe mit 3 C-Atomen ist. Hier hat sich gezeigt, dass das Verhältnis der Längen der Alkylgruppen so ausgewogen ist, dass ein optimales Strukturgefüge gebildet wird, das die Haltbarkeit der erfindungsgemäßen Zubereitung am Applikationsort fördert. Es wird angenommen, dass auf der einen Seite die durch das Silsesquioxanwachs aufgespannte Matrix gerade so weitmaschig ist, dass der erfindungswesentliche Thermoplast und die üblichen kosmetischen Rohstoffkomponenten darin ausreichend stabilisiert werden, so dass sie nicht aus der Zubereitung abwandern oder sich übertragen, dass die Matrix aber auf der anderen Seite so feinmaschig ist, dass die Zubereitung dadurch einen ausreichenden stabilisierenden Effekt erfährt. Insbesondere die langhaftenden Eigenschaften der Zubereitung und die no transfer Eigenschaften sind in dieser Ausführungsform besonders stark ausgeprägt. Es wird angenommen, dass der mindestens eine von Cyclopentadien abgeleitete Einheit, wobei der Thermoplast hydriertes Polydicyclopentadien ist, hier besonders gut in die Struktur und die Kavitäten der von dem Silsesquioxanwachs aufgespannten Matrix einsinken kann, so dass die Elastizität des Gesamtgefüges außerordentlich gesteigert ist, so dass die Zubereitung nicht nur leicht zu applizieren ist, sondern zudem auch lange am Applikationsort haftet und einen gleichmäßigen Film bildet, der selbst nach langer Tragedauer weder brennt noch zu einem spannenden Hautempfinden führt, sondern angenehm weich und geschmeidig bleibt.

Bevorzugt sind Thermoplaste, neben mindestens einem Thermoplasten, der hydriertes Polycyclopentadien ist, die aus nachfolgender Liste ausgewählt werden, wobei die Thermoplaste mit ihrem INCI (International Nomenclature of Cosmetic Ingredients) Namen angegeben sind: Dicyclopentadien/t-Butylcresol Copolymer, Dicyclopentadien/Isopenten/Isopren Copolymer, Dicyclopentadien/Isopenten/ Isopren/Styrol Copolymer, hydriertes Dicyclopentadien/Isopenten/Isopren Copolymer, hydriertes Dicyclopentadien/Isopenten/Isopren/Styrol Copolymer, hydriertes Polycyclopentadien, hydriertes Polydicyclopentadien und Polycyclopentadien oder Mischungen daraus, wobei hydriertes Polydicyclopentadien besonders bevorzugt ist. Durch Verwendung von Copolymeren, also Thermoplasten mit anderen Monomereinheiten als von Cyclopentadien abgeleiteten Einheiten, können die strukturellen Eigenschaften des Gesamtgefüges in der erfindungsgemäßen kosmetischen Zubereitung gezielt eingestellt werden. Dies beeinflusst auch die physikalischen Eigenschaften der Zubereitung, wie Schmelzcharakteristik, Viskosität, Zähigkeit, Brüchigkeit und dergleichen. Der Fachmann kann durch einfache Routineversuche die gewünschten Eigenschaften einstellen. Essentiell ist allerdings, aus denselben Gründen wie vorstehend genannt, die Anwesenheit der von Cyclopentadien abgeleiteten Einheit. Aus oben genannter Liste hat sich hydriertes Polydicyclopentadien als besonders wirksamer, die Elastizität der Zubereitung fördernder, Thermoplast erwiesen. Es wird angenommen, dass dies auf die sehr regelmäßige Struktur zurückzuführen ist. Die Haltbarkeit der erfindungsgemäßen Zubereitung und insbesondere die Wischfestigkeit und der no transfer Charakter konnten hiermit besonders stark erhöht werden, ohne es an Elastizität und gleichzeitig Festigkeit der Zubereitung mangeln zu lassen.

Die erfindungsgemäße Zubereitung kann in Form einer Emulsion, einer Wachs-und/oder Öl- und/oder Silikonmischung oder als Dispersion vorliegen. Besonders deutlich treten die positiven Eigenschaften der erfindungsgemäßen Zubereitung zu tage wenn sie wasserfrei ist. Zwar stabilisiert die erfindungswesentliche Kombination aus Silsesquioxanwachs und Thermoplast jegliche Art von kosmetischer Zubereitung, also auch W/O-Emulsionen, O/W-Emulsionen oder multiple Emulsionen, allerdings ist die Wasserfestigkeit und Wischfestigkeit in wasserfreien Zubereitungen noch einmal deutlich gesteigert.

Die no transfer Eigenschaften der erfindungsgemäßen kosmetischen Zubereitung können durch Zugabe von bei Raumtemperatur flüchtigen Komponenten weiter erhöht werden. Prinzipiell kommt jegliche bei Raumtemperatur flüchtige Komponente für die erfindungsgemäße Zubereitung in Frage. Je höher der Dampfdruck der flüchtigen Komponente, desto schneller verdampft sie nach Applikation und hinterlässt die übrigen, nicht-flüchtigen Komponenten der kosmetischen Zubereitung am Applikationsort, die dann den lang haftenden Film bilden. Geeignete flüchtige Komponenten können ausgewählt werden aus der Gruppe bestehend aus: cyclischen Polydimethylsiloxanen wie z.B. Cyclotetrasiloxan, Cyclopentasiloxan, Cyclohexasiloxan und anderen, flüchtigen linearen Polydimethylsiloxanen, auch Methicone oder Dimethicone genannt, flüchtigen Kohlenwasserstoffen mit 5 bis 16 C-Atomen wie z.B.: Isododecan oder Isohexadecan, oder Mischungen davon. Weitere geeignete flüchtige Rohstoffe sind auch polymere Verbindungen wie solche, die unter dem INCI (International Nomenclature of Cosmetic Ingredients) Polydecene, Polyisobutene, hydrogenated Polyisobutene oder hydrogenated Poly(C6-14 Olefin) erhältlich sind, oder Mischungen daraus, solange diese Verbindungen oder Mischungen bei Raumtemperatur flüchtig sind. Die genannten Rohstoffe verfügen über eine gute Verdunstungsrate bei Raumtemperatur, sind aber auch nicht zu flüchtig, so dass die erfindungsgemäße kosmetische Zubereitung ausreichend lange applizierbar bleibt. In Bezug auf die Menge der flüchtigen Komponente sind keine besonderen Grenzen gesetzt. Die Menge an flüchtiger Komponente kann in Abhängigkeit der gewünschten Applikationsform und der Applikationseigenschaften vom Fachmann leicht bestimmt werden.

Bevorzugt enthält die erfindungsgemäße kosmetische Zubereitung mindestens einen hydrierten polymeren Kohlenwasserstoff. Diese können sowohl linear sein als auch eine verzweigte Struktur aufweisen. Hydrierte polymere Kohlenwasserstoffe haben eine hohe Adhäsion zur Haut und verbessern damit generell die Applikationseigenschaften von für die Haut oder Schleimhäute bestimmten kosmetischen Zubereitungen. Es hat sich aber überraschend herausgestellt, dass hydrierte polymere Kohlenwasserstoffe auch die Elastizität sowie den Glanz der erfindungsgemäßen kosmetischen Zubereitung erhöhen. Es wird davon ausgegangen, dass diese Kohlenwasserstoffe als Bindeglied zwischen den Alkylsilsesquioxaneinheiten fungieren können und aufgrund ihrer hohen Affinität zu dem erfindungswesentlichen Thermoplast nicht störend in das Strukturgefüge eingreifen, sondern eher die Stabilität desselben noch erhöhen, und zwar durch intermolekulare Wechselwirkungen. Dadurch erhöht sich auch die Haftung der Zubereitung auf der Haut, wodurch die no transfer Charakteristik auch auf lange Zeit hin erhalten bleibt. Der gebildete Film haftet stabil am Auftragungsort, wird nicht brüchig und reißt nicht auf, sondern schmiegt sich gleichförmig an die Oberfläche des Applikationsareals an.

Besonders geeignete hydrierte polymere Kohlenwasserstoffe sind dabei ausgewählt aus der Gruppe bestehend aus: Polyisobuten, Polydecen, Polyolefinen mit Einheiten, die 6 bis 14 C-Atomen umfassen, Polybuten, Polyethylen, Polystyrol, Polyisopren, Copolymeren, Terpolymere oder Crosspolymere davon oder Mischungen daraus, wobei die jeweiligen Verbindungen auch teil- oder vollhydriert vorliegen können. Dem Fachmann ist dabei bekannt, welche Monomere bzw. Polymere er miteinander kombinieren kann um gewünschte Eigenschaften der kosmetischen Zubereitung zu fördern.

Die Menge an hydrierten polymeren Kohlenwasserstoffen ist im Einzelnen nicht beschränkt und kann in Abhängigkeit des angestrebten Applikationsprofils leicht durch den Fachmann bestimmt werden.

Des Weiteren enthält die erfindungsgemäße kosmetische Zubereitung bevorzugter Weise noch mindestens einen Polyglycerylester mit 3 bis 10 Glyceryleinheiten. Polyglycerylester werden normalerweise als Emulgatoren in kosmetischen Zubereitungen verwandt. In der erfindungsgemäßen kosmetischen Zubereitung hat sich allerdings gezeigt, dass die Verwendung von mindestens einem Polyglycerylester die Textur- und insbesondere die Farbhomogenität und Farbintensität fördert. Zwar sind die Komponenten der kosmetischen Zubereitung bereits aufgrund der durch die erfindungswesentliche Rohstoffkombination aufgespannten Matrix gleichmäßig in das Gefüge der Zubereitung eingebettet, jedoch hat sich gezeigt, dass durch Verwendung von mindestens einem Polyglycerylester mit 3 bis 10 Glyceryleinheiten bei Benetzung insbesondere von Füllstoffen, Pigmenten und anderen teilpolaren Komponenten, deren gleichmäßige Verteilung noch einmal deutlich gesteigert werden kann. Gerade bei pigmentierten Zubereitungen tritt dieser Effekt besonders deutlich zu Tage. Hier scheint es, als ob der Glycerylester als Anker dient um das Pigment oder den Farbstoff sowohl in der Matrix als auch auf deren Oberfläche zu halten. Dadurch können die Pigmente und Farbstoffe besonders gut mit dem auf sie einfallenden Licht wechselwirken und zeigen ein intensives Farbspektrum. Für diesen sogenannten "Ankereffekt" ist es erforderlich, dass der Glycerylester mindestens drei Glyceryleinheiten aufweist, damit auch ausreichend polare Einheiten für die Anlagerung an Pigmente, Füllstoffe und dergleichen, vorhanden sind. Bei Glycerylestern mit mehr als 10 Glyceryleinheiten ist es hingegen schwierig stabile Zubereitungen zu erhalten, da bei diesen Verbindungen der emulgierende Charakter im Vordergrund zu stehen scheint, was das Matrixgefüge des Silsesquioxanwachses stört, so dass die gewünschten Lagerstabilitäten nicht erhalten werden.

Die Menge an Polyglycerylester ist im Einzelnen nicht beschränkt und kann in Abhängigkeit des angestrebten Applikationsprofils leicht durch den Fachmann bestimmt werden.

Die Glycerylester sind im Einzelnen nicht beschränkt. Als besonders vorteilhaft haben sich Glycerylester erwiesen, die aus nachfolgender Liste ausgewählt sind, wobei die Glycerylester mit ihrem INCI (International Nomenclature of Cosmetic Ingredients) Namen angegeben sind: Aprikosenkernöl Polyglyceryl-6 Ester, Aprikosenkernöl Polyglyceryl-10 Ester, Babassuöl Polyglyceryl-4 Esters, Babassuöl Polyglyceryl-6 Ester, Bis-Butyldimethicone Polyglyceryl-3, Borretschsamenöl Polyglyceryl-6 Ester, Candelilla/Jojoba/Reisschalen Polyglyceryl-3 Ester, Kakaobutter Polyglyceryl-6 Ester, Kokosnussöl Polyglyceryl-6 Ester, Kaffesamenöl Polyglyceryl-6 Ester, Diisostearoyl Polyglyceryl-3 Dimer Dilinoleat, Glyceryl/Polyglyceryl-6 Isostearat/Behenat Ester, Isopolyglyceryl-3 Dimethicon, Isopolyglyceryl-3 Dimethiconol, Lauryl Polyglyceryl-6 Cetearyl Glycol Ether, Macadamiasamenöl Polyglyceryl-6 Ester Behenat, Olivenöl Polyglyceryl-4 Ester, Olivenöl Polyglyceryl-6 Ester, Polyglyceryl-3 Bienenwachs, Polyglyceryl-6 Behenat, Polyglyceryl-10 Behenat/Eicosadioat, Polyglyceryl-8 C12-20 Säure Ester, Polyglyceryl-3 Caprat, Polyglyceryl-4 Caprat, Polyglyceryl-5 Caprat, Polyglyceryl-6 Caprat, Polyglyceryl-10 Caprat, Polyglyceryl-3 Caprylat, Polyglyceryl-4 Caprylat, Polyglyceryl-6 Caprylat, Polyglyceryl-10 Caprylat, Polyglyceryl-3 Cocoat, Polyglyceryl-4 Cocoat, Polyglyceryl-8 Decaerucat/Decaisostearat/Decaricinoleat, Polyglyceryl-10 Decaethylhexanoat, Polyglyceryl-10 Decahydroxystearat, Polyglyceryl-10 Decaisostearat, Polyglyceryl-10 Decastearat, Polyglyceryl-3 Dicaprat, Polyglyceryl-3 Diisostearat, Polyglyceryl-6 Diisostearat, Polyglyceryl-10 Diisostearat, Polyglyceryl-4 Dilaurat, Polyglyceryl-5 Dilaurat, Polyglyceryl-10 Dimyristat, Polyglyceryl-3 Dioleat, Polyglyceryl-5 Dioleat, Polyglyceryl-6 Dioleat, Polyglyceryl-10 Dioleat, Polyglyceryl-6 Dipalmitat, Polyglyceryl-10 Dipalmitat, Polyglyceryl-3 Disiloxane Dimethicon, Polyglyceryl-3 Distearat, Polyglyceryl-10 Hexaerucat,Polyglyceryl-10 Hexaisostearat, Polyglyceryl-6 Hexaoleat, Polyglyceryl-10 Hexaoleat, Polyglyceryl-5 Hexastearat, Polyglyceryl-3 Isostearat, Polyglyceryl-4 Isostearat, Polyglyceryl-5 Isostearat, Polyglyceryl-6 Isostearat, Polyglyceryl-4 Isostearat/Laurat, Polyglyceryl-3/Lauryl Polydimethylsiloxyethyl Dimethicone Crosspolymer, Polyglyceryl-10 Linoleat, Polyglyceryl-3 Methylglucose Distearat, Polyglyceryl-3 Myristat, Polyglyceryl-5 Myristat, Polyglyceryl-3 Oleat, Polyglyceryl-4 Oleat, Polyglyceryl-10 Oleat, Polyglyceryl-3 Palmitat, Polyglyceryl-6 Palmitat, Polyglyceryl-10 Palmitat, Polyglyceryl-10 Pentaisostearat, Polyglyceryl-10 Pentalaurat, Polyglyceryl-5 Pentamyristat, Polyglyceryl-3 Pentaricinoleat, Polyglyceryl-6 Pentaricinoleat, Polyglyceryl-4 Pentastearat, Polyglyceryl-6 Pentastearat, Polyglyceryl-3 Polydimethylsiloxyethyl Dimethicon, Polyglyceryl-6 Polyhydroxystearat, Polyglyceryl-4 Diisostearate/Polyhydroxystearat /Sebacat, Polyglyceryl-3 Polyricinoleat, Polyglyceryl-4 Polyricinoleat, Polyglyceryl-6 Sesquiisostearat, Polyglyceryl-6 Sesquistearat, Polyglyceryl-3 Stearat, Polyglyceryl-4 Stearat, Polyglyceryl-6 Tetrabehenat, Polyglyceryl-6 Tetracaprylat, Polyglyceryl-5 Tribehenat, Polyglyceryl-3 Triisostearat, Polyglyceryl-5 Trimyristat, Polyglyceryl-4 Tristearat, Polyglyceryl-5 Tristearat, Distelöl Polyglyceryl-6 Ester, Sesamöl Polyglyceryl-6 Ester, Shea Butter Polyglyceryl-6 Ester, Sojabohnenöl Polyglyceryl-6 Ester, Sonnenblumensamenöl Polyglyceryl-6 Ester oder Mischungen daraus.

Die erfindungsgemäße Zubereitung kann bevorzugter Weise auch Füllstoffe enthalten, wie zum Beispiel Polymethylsilsesquioxan, Dimethicone/Vinyldimethicone Crosspolymer, Dimethicon/Bis-Isobutyl PPG-20 Crosspolymer, Dimethicone Crosspolymer, Mica, Silica, Nylon-12, PMMA, Bornitrid, Polyethylen, HDI/Trimethylol Hexyllacton Crosspolymer, Magnesiumstearat, Zinkoxid oder Mischungen daraus (die vorstehenden Bezeichnungen gemäß INCI - International Nomenclature of Cosmetic Ingredients). Mit Hilfe dieser Füllstoffe lässt sich das Applikationsprofil der Zubereitung variieren von weich gleitend bis trocken stumpf. Dem Fachmann sind gängige Füllstoffe bekannt, jedoch haben sich die oben benannten als am verträglichsten mit der erfindungsgemäßen Zubereitung herausgestellt, so dass gerade die Langzeitstabilität bei erhöhter und auch bei tiefer Temperatur nicht nachteilig beeinflusst wird. So sind die erfindungsgemäßen Zubereitungen bei Temperaturen von -10 °C bis 45 °C über vier Wochen stabil, das heißt, es sind nach Lagerung weder optisch noch sensorisch Unterschiede zu den entsprechenden nicht belasteten Zubereitungen feststellbar.

Die Menge an Füllstoff ist im Einzelnen nicht beschränkt und kann in Abhängigkeit des angestrebten Applikationsprofils leicht durch den Fachmann bestimmt werden.

Weiterhin vorteilhaft ist es, wenn die Zubereitung sogenannte Filmbildner enthält. Filmbildner im Sinne der Erfindung sind solche Verbindungen, die eine gewisse Adhäsion zu Applikationsareal, wie zum Beispiel der Haut haben und dort dazu beitragen die erfindungsgemäße Zubereitung auf der Oberfläche der Haut zu stabilisieren. Damit wird die Haftung der Zubereitung auf der Haut erhöht, insbesondere Langzeithaftung der Zubereitung. Auch die no transfer Charakteristik der Zubereitung wird durch Zugabe von Filmbildnern erhöht. Die erfindungsgemäße Zubereitung ist damit noch länger, also über mehrere Stunden bis zu einem Tag am Applikationsort fixiert, ohne von dort abzuwandern oder sich zu übertragen. Bevorzugt sind die Filmbildner im Sinne der Erfindung aus Polymeren ausgewählt, die für diese Zwecke bekannt sind, wie Polyurethanpolymere, Acrylsäure oder Acrylat basierte Polymere, silikonbasierte Polymere, Copolymere oder Crosspolymere davon, sowie Mischungen daraus. Die Filmbildner können in den üblichen verwendeten Mengen eingesetzt werden, die der Fachmann leicht durch Routineversuche herausfinden kann. Als besonders geeigneter Filmbildner hat sich ein Polymer herausgestellt, das unter dem INCI Acrylates/Dimethicone Copolymer gelistet wird. Dieses lässt sich einfach und homogen in die erfindungsgemäße Zubereitung einarbeiten und bildet nach Verdunsten aller flüchtigen Komponenten am Applikationsort einen stabilen aber elastischen Film, der über mehrere Stunden stabil ist, nicht aufreißt und die in der Zubereitung enthaltenen Komponenten dauerhaft einschließt, so dass ein "Ausbluten" oder Abwandern einzelner Komponenten unterbunden wird. Der no transfer Effekt dieser erfindungsgemäßen Zubereitung wird deutlich erhöht.

Die erfindungsgemäße kosmetische Zubereitung kann ferner weitere gängige, für kosmetische Zubereitungen geeignete Komponenten enthalten, wie zum Beispiel Pigmente, Farbstoffe, Antioxidantien, Konservierungsstoffe, Aromen, Puffer, Öle, Weichmacher, Pflegestoffe und andere, die in den üblichen Mengen eingesetzt werden.

Des Weiteren kann die erfindungsgemäße kosmetische Zubereitung in flüssiger, halbfester, also cremiger oder pastöser oder fester Form vorliegen und kann nach Abfüllung in geeignete Applikationsgefäße leicht wieder daraus entnommen werden. Es hat sich herausgestellt, dass die besonderen Trageeigenschaften nicht nur in pastösen oder flüssigen Zubereitungen verwirklicht werden können, sondern auch in festen Massen, die zu Minen formbar sind, die dann als kosmetische Stifte angeboten werden können. Kosmetische Stifte haben dabei den Vorteil, dass sie handlich sind und jederzeit leicht angewendet werden können. Die erfindungsgemäße Zubereitung ist in fester Form mechanisch so belastbar, dass aus ihr Minen geformt werden können, die sogar eine ausreichende Festigkeit haben, um freistehend in Drehmechaniken eingesetzt werden zu können.

Verfahren zur Herstellung von Minen sind dem Fachmann wohlbekannt und die bekannten Verfahren eignen sich zur Verarbeitung der erfindungsgemäßen Massen. So kann die erfindungsgemäße Zubereitung durch Gießen oder durch Verformen der Masse mittels Anwendung von Druck geformt werden. Die erhaltenen Minen können entweder in Drehmechaniken eingesetzt oder zu Stiften verarbeitet werden, z.B. zu Holzstiften. Ebenso ist es möglich die Masse direkt in eine Mechanik oder eine Hülse für einen Stift zu gießen. Aufgrund ihrer guten Eigenschaften können die aus der erfindungsgemäßen Masse geformten Minen in vielfältiger Form vorliegen, wobei der Minendurchmesser je nach Anwendungsgebiet eingestellt werden kann. Geeignete Minendurchmesser bewegen sich im Bereich von 1 bis 20 mm, bevorzugt 3 bis 10 mm.

Als weiterer Vorteil der erfindungsgemäßen Zubereitung hat sich gezeigt, dass sie besonders gut zu freistehenden Minen formbar ist, die über eine ausgezeichnete Stabilität, also Bruchfestigkeit, Lagerstabilität, Anwendungsstabilität und dergleichen, verfügen, ohne dass sich deren Stabilität nachteilig auf die Applikationseigenschaften auswirkt. Freistehende Minen, die zumeist in Drehmechaniken eingesetzt werden, um einen applikationsfähigen Stift zu ergeben, sind normalerweise besonders anfällig im Hinblick auf mechanische Belastungen. Die erfindungsgemäße Zubereitung jedoch weist aufgrund ihrer stabilen inneren Struktur eine ausgezeichnete Stabilität auf und ist daher besonders gut für die Herstellung freistehender Minen verwendbar.

Die Erfindung betrifft zudem die Verwendung der genannten kosmetischen Zubereitung zur Herstellung eines Kosmetikstifts.

Die erfindungsgemäße Zubereitung eignet sich besonders gut zur Herstellung eines Kosmetikstifts. Ein solcher Kosmetikstift kann ein Eyeliner, Eyeshadow, Eyebrow, Lipliner, Lipstick, Wimperntusche, Tattoo, Wangenrouge oder eine Abdeckmasse wie ein Abdeckstift sein.

### Beispiele:

Soweit nicht anders angegeben, beziehen sich die Mengenangaben der einzelnen Komponenten auf Gewichtsprozent in Bezug auf das Gesamtgewicht der Zusammensetzung.

Soweit nicht anders angegeben werden die einzelnen Komponenten mit ihrem INCI (International Nomenclature of Cosmetic Ingredients) angegeben.

### Beispiel 1: Eyeliner in Stiftform

| Komponente | Gewichts.-% |
|---|---|
| Polyethylene | 13 |
| Synthetic Wax | 8 |
| Hydrogenated Polydiclyclopentadiene | 7 |
| Polyglyceryl-10 Pentaisostearate | 3 |
| C30-45 Alkyldimethylsilyl Polypropylsilsesquioxane | 6 |
| Antioxidant | 0,2 |
| Polymethyl Methacrylate | 5 |
| Isoparaffin | 22,8 |
| Pigments | 35 |

### Zubereitung:

In einem geeigneten Behälter wurden alle Komponenten bis auf das Isoparaffin und die Pigmente bis zur Homogenität unter Rühren aufgeschmolzen. Anschließend wurden die Pigmente zugefügt und die Masse homogenisiert. Anschließend wurde die pigmenthaltige Masse erneut aufgeschmolzen, das Isoparaffin zugegeben und die Masse heiß in eine entsprechende Stiftform gegossen.

Nach dem Abkühlen konnte aus der Form eine Mine mit einem Durchmesser von 4 mm entnommen werden, die in eine entsprechende Mechanik eingefügt werden konnte.

Die Masse zeichnete sich durch eine angenehm weiche, leicht cremige Applikation am oberen wie auch am unteren Augenlid aus. Die Farbverteilung war homogen. Die Zubereitung haftete mehr als 8 Stunden am Applikationsort und übertrug sich nicht auf das obere Augenlid.

Die Zubereitung war zudem wasserfest, das heißt, dass nach dem Abtrocknen und nach anschließendem Umspülen der applizierten Fläche mit einem 40 °C warmen Wasser für 10 Minuten optisch mit bloßem Auge keine Veränderung der applizierten Zubereitung feststellbar war.

Ferner konnte bei Lagerung des Stiftes über 4 Wochen bei -10 °C, 45 °C und im Vergleich dazu bei 25 °C (Raumtemperatur) weder optisch noch sensorisch eine Veränderung zu entsprechenden nicht belasteten Stiften beobachtet werden.

### Beispiel 2: Lippenstift

| Komponente | Gewichts.-% |
|---|---|
| Candelilla Wax | 10 |
| Polyethylene | 6 |
| Hydrogenated Polydiclyclopentadiene | 7,5 |
| C30-45 Alkyldimethylsilyl Polypropylsilsesquioxane | 7,5 |
| Antioxidant | 0,3 |
| Polyglyceryl-4 Diisostearate/Polyhydroxystearate | 4,2 |
| Acrylates/Dimethicone Copolymer | 5 |
| Dimethicone | 11,3 |
| Polybutene | 6,2 |
| Isododecane | 15,1 |
| Sheabutter | 0,8 |
| Pigments | 22 |
| Polymethylsilsesquioxane | 4,1 |

### Zubereitung:

In einem geeigneten Behälter wurden alle Komponenten bis auf das Isododecane und die Pigmente bis zur Homogenität unter Rühren aufgeschmolzen. Anschließend wurden die Pigmente zugefügt und die Masse homogenisiert. Anschließend wurde die pigmenthaltige Masse erneut aufgeschmolzen, das Isododecane zugegeben und die Masse heiß in eine entsprechende Form gegossen.

Nach dem Abkühlen konnte aus der Form eine Mine mit einem Durchmesser von 8 mm entnommen werden, die in eine entsprechende Lippenstiftmechanik eingefügt werden konnte.

Die Masse zeichnete sich durch eine leichte, cremige Applikation aus und ließ sich geschmeidig auf den Lippen verteilen. Die Farbverteilung war homogen und deckend. Die Zubereitung haftete mehr als 8 Stunden am Applikationsort und übertrug sich nicht auf mit den Lippen in Berührung kommende Zigaretten, Tassen, Taschentücher oder Hautpartien.

Die Zubereitung war zudem wasserfest, das heißt, dass nach dem Abtrocknen und nach anschließendem Umspülen der applizierten Fläche mit einem 40 °C warmen Wasser für 10 Minuten optisch mit bloßem Auge keine Veränderung der applizierten Zubereitung feststellbar war.

Ferner konnte bei Lagerung des Stiftes über 4 Wochen bei -10 °C, 45 °C und im Vergleich dazu bei 25 °C (Raumtemperatur) weder optisch noch sensorisch eine Veränderung zu entsprechenden nicht belasteten Stiften beobachtet werden.

### Beispiel 3: Eyeshadow

| Komponente | Gewichts.-% |
|---|---|
| Stearyl Dimethicone | 9 |
| Polyethylene | 8,2 |
| C20-40 Alcohols and Polyethylene | 1,0 |
| Hydrogenated Polydiclyclopentadiene | 7,8 |
| C30-45 Alkyldimethylsilyl Polypropylsilsesquioxane | 10,6 |
| Antioxidant | 0,2 |
| Squalane | 6,0 |
| Microcristalline Wax | 2,0 |
| Hydrogenated Polydecene | 4,4 |
| Hydrogenated Polyisobutene | 4,3 |
| Isododecane | 20,9 |
| Hydrogenated (C6-14 Olefin)Polymers | 5,8 |
| Pigments/Füllstoffe | 17,6 |
| HDI/Trimethylol Hexyllactone Crosspoplymer | 2,2 |

### Zubereitung:

In einem geeigneten Behälter wurden alle Komponenten bis auf das Isododecane und die Pigmente bis zur Homogenität unter Rühren aufgeschmolzen. Anschließend wurden die Pigmente zugefügt und die Masse homogenisiert. Anschließend wurde die pigmenthaltige Masse erneut aufgeschmolzen, das Isododecane zugegeben und die Masse heiß in eine Tube gefüllt.

Nach dem Abkühlen konnte die Masse einfach aus der Tube auf einen Finger oder ein Applikationsgerät appliziert werden.

Die Masse zeichnete sich durch eine leichte, weiche Applikation aus und ließ sich einfach ohne viel Druck aufzubringen auf dem Augenlid verteilen. Die Farbverteilung war homogen und deckend. Die Zubereitung haftete mehr als 8 Stunden am Applikationsort und übertrug sich nicht auf andere Hautpartien.

Die Zubereitung war zudem wasserfest, das heißt, dass nach dem Abtrocknen und nach anschließendem Umspülen der applizierten Fläche mit einem 40 °C warmen Wasser für 10 Minuten optisch mit bloßem Auge keine Veränderung der applizierten Zubereitung feststellbar war.

Ferner konnte bei Lagerung der Masse über 4 Wochen bei -10 °C, 45 °C und im Vergleich dazu bei 25 °C (Raumtemperatur) weder optisch noch sensorisch eine Veränderung zu entsprechender nicht belasteter Masse beobachtet werden.

### Beispiel 4: Concealer

| Komponente | Gewichts.-% |
|---|---|
| Hydrogenated Jojoba Oil | 10,2 |
| Phenyl Trimethicone | 4,5 |
| Polyglyceryl-3 Diisostearate | 3,0 |
| Hydrogenated Polyclyclopentadiene | 4,0 |
| C30-45 Alkyldimethylsilyl Polypropylsilsesquioxane | 8,1 |
| Preservative | 0,8 |
| Hydrogenated Polyisobutene | 3,3 |
| Dimethicone | 14,2 |
| Cyclopentasiloxane | 22,8 |
| Nylon-12 | 14,0 |
| Pigments | 8,1 |
| Silica | 2,2 |
| Boron Nitride | 4,5 |
| Antioxidant | 0,3 |

### Zubereitung:

In einem geeigneten Behälter wurden alle Komponenten bis auf das Cyclopentasiloxane und die Pigmente bis zur Homogenität unter Rühren aufgeschmolzen. Anschließend wurden die Pigmente zugefügt und die Masse homogenisiert. Anschließend wurde die pigmenthaltige Masse erneut aufgeschmolzen, das Cyclopentasiloxane zugegeben und die Masse heiß in eine entsprechende Stiftform gegossen.

Nach dem Abkühlen konnte aus der Form eine Mine mit einem Durchmesser von 6 mm entnommen werden, die in eine entsprechende Stiftmechanik eingefügt werden konnte.

Die Masse zeichnete sich durch eine leicht pudrige, trocken weiche Applikation aus und ließ sich einfach ohne viel Druck aufzubringen auf der Haut verteilen. Die Farbverteilung war homogen und semideckend. Die Zubereitung haftete mehr als 8 Stunden am Applikationsort und übertrug sich nicht auf andere Hautpartien oder damit in Berührung kommende Textilien.

Die Zubereitung war zudem wasserfest, das heißt, dass nach dem Abtrocknen und nach anschließendem Umspülen der applizierten Fläche mit einem 40 °C warmen Wasser für 10 Minuten optisch mit bloßem Auge keine Veränderung der applizierten Zubereitung feststellbar war.

Ferner konnte bei Lagerung des Stiftes über 4 Wochen bei -10 °C, 45 °C und im Vergleich dazu bei 25 °C (Raumtemperatur) weder optisch noch sensorisch eine Veränderung zu entsprechenden nicht belasteten Stiften beobachtet werden.

## Patentansprüche

1. Kosmetische Zubereitung enthaltend mindestens ein Silsesquioxanwachs der Formel
(R₂R'SiO_{1/2})ₓ(R"SiO_{3/2})_{y}
wobei R jeweils eine Alkylgruppe mit 1 bis 8 C-Atomen ist, R' eine einwertige Alkylgruppe mit 9 bis 46 C-Atomen, R" eine einwertige Alkylgruppe mit 1 bis 8 C-Atomen oder eine Arylgruppe ist und wobei x und y Werte zwischen 0,05 und 0,95 annehmen können, und mindestens einen Thermoplast mit mindestens einer von Cyclopentadien abgeleiteten Einheit wobei der Thermoplast hydriertes Polydicyclopentadien ist.

2. Kosmetische Zubereitung nach Anspruch 1, **dadurch gekennzeichnet, dass** in dem Silsesquioxanwachs R jeweils eine Methylgruppe, R' eine Alkylgruppe mit 30 bis 46 C-Atomen und R" eine Alkylgruppe mit 3 C-Atomen ist.

3. Kosmetische Zubereitung nach einem der vorhergehenden Ansprüche, **dadurch gekennzeichnet, dass** die Zubereitung wasserfrei ist.

4. Kosmetische Zubereitung nach einem der vorhergehenden Ansprüche, **dadurch gekennzeichnet, dass** die Zubereitung mindestens eine bei Raumtemperatur flüchtige Komponente enthält.

5. Kosmetische Zubereitung nach Anspruch 4, **dadurch gekennzeichnet, dass** die flüchtige Komponente ausgewählt ist aus der Gruppe bestehend aus: cyclischen Polydimethylsiloxanen wie z.B. Cyclotetrasiloxan, Cyclopentasiloxan, Cyclohexasiloxan, linearen Polydimethylsiloxanen, flüchtigen Kohlenwasserstoffen mit 5 bis 16 C-Atomen wie z.B.: Isododecan oder Isohexadecan; Polydecene, Polyisobutene, hydrogenated Polyisobutene oder hydrogenated Poly(C6-14 Olefin) oder Mischungen davon.

6. Kosmetische Zubereitung nach einem der vorhergehenden Ansprüche, **dadurch gekennzeichnet, dass** die Zubereitung mindestens einen hydrierten polymeren Kohlenwasserstoff enthält.

7. Kosmetische Zubereitung nach Anspruch 6, **dadurch gekennzeichnet, dass** der hydrierte, polymere Kohlenwasserstoff ausgewählt ist aus der Gruppe bestehend aus: Polyisobuten, Polydecen, Polyolefinen mit Einheiten, die 6 bis 14 C-Atomen umfassen, Polybuten, Polyethylen, Polystyrol, Polyisopren, Copolymeren davon oder Mischungen daraus.

8. Kosmetische Zubereitung nach einem der vorhergehenden Ansprüche, **dadurch gekennzeichnet, dass** die Zubereitung mindestens einen Polyglycerylester enthält.

9. Kosmetische Zubereitung nach Anspruch 8, **dadurch gekennzeichnet, dass** der Polyglycerylester 3 bis 10 Glyceryleinheiten aufweist.

10. Kosmetische Zubereitung nach Anspruch 8 oder 9, **dadurch gekennzeichnet, dass** der Glycerylester ausgewählt ist aus der Gruppe bestehend aus:
Aprikosenkernöl Polyglyceryl-6 Ester, Aprikosenkernöl Polyglyceryl-10 Ester, Babassuöl Polyglyceryl-4 Esters, Babassuöl Polyglyceryl-6 Ester, Bis-Butyldimethicone Polyglyceryl-3, Borretschsamenöl Polyglyceryl-6 Ester, Candelilla/Jojoba/Reisschalen Polyglyceryl-3 Ester, Kakaobutter Polyglyceryl-6 Ester, Kokosnussöl Polyglyceryl-6 Ester, Kaffesamenöl Polyglyceryl-6 Ester, Diisostearoyl Polyglyceryl-3 Dimer Dilinoleat, Glyceryl/Polyglyceryl-6 Isostearat/Behenat Ester, Isopolyglyceryl-3 Dimethicon, Isopolyglyceryl-3 Dimethiconol, Lauryl Polyglyceryl-6 Cetearyl Glycol Ether, Macadamiasamenöl Polyglyceryl-6 Ester Behenat, Olivenöl Polyglyceryl-4 Ester, Olivenöl Polyglyceryl-6 Ester, Polyglyceryl-3 Bienenwachs, Polyglyceryl-6 Behenat, Polyglyceryl-10 Behenat/Eicosadioat, Polyglyceryl-8 C₁₂-₂₀ Säure Ester, Polyglyceryl-3 Caprat, Polyglyceryl-4 Caprat, Polyglyceryl-5 Caprat, Polyglyceryl-6 Caprat, Polyglyceryl-10 Caprat, Polyglyceryl-3 Caprylat, Polyglyceryl-4 Caprylat, Polyglyceryl-6 Caprylat, Polyglyceryl-10 Caprylat, Polyglyceryl-3 Cocoat, Polyglyceryl-4 Cocoat, Polyglyceryl-8 Decaerucat/Decaisostearat/Decaricinoleat, Polyglyceryl-10 Decaethylhexanoat, Polyglyceryl-10 Decahydroxystearat, Polyglyceryl-10 Decaisostearat, Polyglyceryl-10 Decastearat, Polyglyceryl-3 Dicaprat, Polyglyceryl-3 Diisostearat, Polyglyceryl-6 Diisostearat, Polyglyceryl-10 Diisostearate, Polyglyceryl-4 Dilaurat, Polyglyceryl-5 Dilaurat, Polyglyceryl-10 Dimyristat, Polyglyceryl-3 Dioleat, Polyglyceryl-5 Dioleat, Polyglyceryl-6 Dioleat, Polyglyceryl-10 Dioleat, Polyglyceryl-6 Dipalmitat, Polyglyceryl-10 Dipalmitat, Polyglyceryl-3 Disiloxane Dimethicon, Polyglyceryl-3 Distearat, Polyglyceryl-10 Hexaerucat,Polyglyceryl-10 Hexaisostearat, Polyglyceryl-6 Hexaoleat, Polyglyceryl-10 Hexaoleat, Polyglyceryl-5 Hexastearat, Polyglyceryl-3 Isostearat, Polyglyceryl-4 Isostearat, Polyglyceryl-5 Isostearat, Polyglyceryl-6 Isostearat, Polyglyceryl-4 Isostearat/Laurat, Polyglyceryl-3/Lauryl Polydimethylsiloxyethyl Dimethicone Crosspolymer, Polyglyceryl-10 Linoleat, Polyglyceryl-3 Methylglucose Distearat, Polyglyceryl-3 Myristat, Polyglyceryl-5 Myristat, Polyglyceryl-3 Oleat, Polyglyceryl-4 Oleat, Polyglyceryl-10 Oleat, Polyglyceryl-3 Palmitat, Polyglyceryl-6 Palmitat, Polyglyceryl-10 Palmitat, Polyglyceryl-10 Pentaisostearat, Polyglyceryl-10 Pentalaurat, Polyglyceryl-5 Pentamyristat, Polyglyceryl-3 Pentaricinoleat, Polyglyceryl-6 Pentaricinoleat, Polyglyceryl-4 Pentastearat, Polyglyceryl-6 Pentastearat, Polyglyceryl-3 Polydimethylsiloxyethyl Dimethicon, Polyglyceryl-6 Polyhydroxystearat, Polyglyceryl-4 Diisostearate /Polyhydroxystearat/Sebacat, Polyglyceryl-3 Polyricinoleat, Polyglyceryl-4 Polyricinoleat, Polyglyceryl-6 Sesquiisostearat, Polyglyceryl-6 Sesquistearat, Polyglyceryl-3 Stearat, Polyglyceryl-4 Stearat, Polyglyceryl-6 Tetrabehenat, Polyglyceryl-6 Tetracaprylat, Polyglyceryl-5 Tribehenat, Polyglyceryl-3 Triisostearat, Polyglyceryl-5 Trimyristat, Polyglyceryl-4 Tristearat, Polyglyceryl-5 Tristearat, Distelöl Polyglyceryl-6 Ester, Sesamöl Polyglyceryl-6 Ester, Shea Butter Polyglyceryl-6 Ester, Sojabohnenöl Polyglyceryl-6 Ester, Sonnenblumensamenöl Polyglyceryl-6 Ester oder Mischungen daraus.

11. Kosmetische Zubereitung nach einem der vorhergehenden Ansprüche, **dadurch gekennzeichnet, dass** die Zubereitung mindestens einen Füllstoff enthält, ausgewählt aus der Gruppe bestehend aus: Polymethylsilsesquioxan, Dimethicone/Vinyldimethicone Crosspolymer, Dimethicon/Bis-Isobutyl PPG-20 Crosspolymer, Dimethicone Crosspolymer, Mica, Silica, Nylon-12, PMMA, Bornitrid, Polyethylen, HDI/Trimethylol Hexyllacton Crosspolymer, Magnesiumstearat, Zinkoxid oder Mischungen daraus.

12. Kosmetische Zubereitung nach einem der vorhergehenden Ansprüche, **dadurch gekennzeichnet, dass** die Zubereitung zu einer Mine formbar ist.

13. Verwendung einer kosmetischen Zubereitung nach einem der Ansprüche 1 bis 12 als Eyeliner, Eyeshadow, Eyebrow, Tattoo, Lipliner, Lipstick, Wimperntusche, Wangenrouge oder Abdeckmasse.

14. Verwendung einer kosmetischen Zubereitung nach einem der Ansprüche 1 bis 12 zur Herstellung eines Kosmetikstifts.

## Claims

1. A cosmetic preparation containing at least one silsesquioxane wax of the formula
(R₂R'SiO_{1/2})ₓ(R"SiO_{3/2})_{y}
wherein R is in each case an alkyl group having 1 to 8 C-atoms, R' is a monovalent alkyl group having 9 to 46 C-atoms, R" is a monovalent alkyl group having 1 to 8 C-atoms or an aryl group, and wherein x and y can assume values between 0.05 and 0.95, and at least one thermoplastic having at least one unit derived from cyclopentadiene, wherein the thermoplastic is hydrated polydicyclopentadiene.

2. A cosmetic preparation according to claim 1, **characterised in that** in the silsesquioxane wax R is in each case a methyl group, R' is an alkyl group having 30 to 46 C-atoms, and R" is an alkyl group having 3 C-atoms.

3. A cosmetic preparation according to one of the preceding claims, **characterised in that** the preparation is water-free.

4. A cosmetic preparation according to one of the preceding claims, **characterised in that** the preparation contains at least one component that is volatile at ambient temperature.

5. A cosmetic preparation according to claim 4, **characterised in that** the volatile component is selected from the group consisting of: cyclic polydimethylsiloxanes, such as, for example, cyclotetrasiloxane, cyclopentasiloxane, cyclohexasiloxane, linear polydimethylsiloxanes, volatile hydrocarbons having 5 to 16 C-atoms, such as, for example: isododecane or isohexadecane; polydecenes, polyisobutenes, hydrogenated polyisobutenes or hydrogenated poly(C6-14 olefin) or mixtures thereof.

6. A cosmetic preparation according to one of the preceding claims, **characterised in that** the preparation contains at least one hydrated polymeric hydrocarbon.

7. A cosmetic preparation according to claim 6, **characterised in that** the hydrated polymeric hydrocarbon is selected from the group consisting of:
polyisobutene, polydecene, polyolefins with units that comprise 6 to 14 C-atoms, polybutene, polyethylene, polystyrene, polyisoprene, copolymers thereof or mixtures thereof.

8. A cosmetic preparation according to one of the preceding claims, **characterised in that** the preparation contains at least one polyglyceryl ester.

9. A cosmetic preparation according to claim 8, **characterised in that** the polyglyceryl ester has 3 to 10 glyceryl units

10. A cosmetic preparation according to claim 8 or 9, **characterised in that** the glyceryl ester is selected from the group consisting of:
apricot kernel oil polyglyceryl-6 ester, apricot kernel oil polyglyceryl-10 ester, babassu oil polyglyceryl-4 esters, babassu oil polyglyceryl-6 ester, bis-butyldimethicone polyglyceryl-3, borage seed oil polyglyceryl-6 ester, candelilla/jojoba/rice husks polyglyceryl-3 ester, cocoa butter polyglyceryl-6 ester, coconut oil polyglyceryl-6 ester, coffee seed oil polyglyceryl-6 ester, diisostearoyl polyglyceryl-3 dimer dilinoleate, glyceryl/polyglyceryl-6 isostearate/behenate ester, isopolyglyceryl-3 dimethicone, isopolyglyceryl-3 dimethiconol, lauryl polyglyceryl-6 cetearyl glycol ether, macadamia seed oil polyglyceryl-6 ester behenate, olive oil polyglyceryl-4 ester, olive oil polyglyceryl-6 ester, polyglyceryl-3 beeswax, polyglyceryl-6 behenate, polyglyceryl-10 behenate/eicosadioate, polyglyceryl-8 C₁₂₋₂₀ acid ester, polyglyceryl-3 caprate, polyglyceryl-4 caprate, polyglyceryl-5 caprate, polyglyceryl-6 caprate, polyglyceryl-10 caprate, polyglyceryl-3 caprylate, polyglyceryl-4 caprylate, polyglyceryl-6 caprylate, polyglyceryl-10 caprylate, polyglyceryl-3 cocoate, polyglyceryl-4 cocoate, polyglyceryl-8 decaerucate/decaisostearate/decaricinoleate, polyglyceryl-10 decaethylhexanoate, polyglyceryl-10 decahydroxystearate, polyglyceryl-10 decaisostearate, polyglyceryl-10 decastearate, polyglyceryl-3 dicaprate, polyglyceryl-3 diisostearate, polyglyceryl-6 diisostearate, polyglyceryl-10 diisostearate, polyglyceryl-4 dilaurate, polyglyceryl-5 dilaurate, polyglyceryl-10 dimyristate, polyglyceryl-3 dioleate, polyglyceryl-5 dioleate, polyglyceryl-6 dioleate, polyglyceryl-10 dioleate, polyglyceryl-6 dipalmitate, polyglyceryl-10 dipalmitate, polyglyceryl-3 disiloxane dimethicone, polyglyceryl-3 distearate, polyglyceryl-10 hexaerucate, polyglyceryl-10 hexaisostearate, polyglyceryl-6 hexaoleate, polyglyceryl-10 hexaoleate, polyglyceryl-5 hexastearate, polyglyceryl-3 isostearate, polyglyceryl-4 isostearate, polyglyceryl-5 isostearate, polyglyceryl-6 isostearate, polyglyceryl-4 isostearate/laurate, polyglyceryl-3/lauryl polydimethylsiloxyethyl dimethicone crosspolymer, polyglyceryl-10 linoleate, polyglyceryl-3 methylglucose distearate, polyglyceryl-3 myristate, polyglyceryl-5 myristate, polyglyceryl-3 oleate, polyglyceryl-4 oleate, polyglyceryl-10 oleate, polyglyceryl-3 palmitate, polyglyceryl-6 palmitate, polyglyceryl-10 palmitate, polyglyceryl-10 pentaisostearate, polyglyceryl-10 pentalaurate, polyglyceryl-5 pentamyristate, polyglyceryl-3 pentaricinoleate, polyglyceryl-6 pentaricinoleate, polyglyceryl-4 pentastearate, polyglyceryl-6 pentastearate, polyglyceryl-3 polydimethylsiloxyethyl dimethicone, polyglyceryl-6 polyhydroxystearate, polyglyceryl-4 diisostearate/polyhydroxystearate/sebacate, polyglyceryl-3 polyricinoleate, polyglyceryl-4 polyricinoleate, polyglyceryl-6 sesquiisostearate, polyglyceryl-6 sesquistearate, polyglyceryl-3 stearate, polyglyceryl-4 stearate, polyglyceryl-6 tetrabehenate, polyglyceryl-6 tetracaprylate, polyglyceryl-5 tribehenate, polyglyceryl-3 triisostearate, polyglyceryl-5 trimyristate, polyglyceryl-4 tristearate, polyglyceryl-5 tristearate, thistle oil polyglyceryl-6 ester, sesame oil polyglyceryl-6 ester, shea butter polyglyceryl-6 ester, soya bean oil polygylceryl-6 ester, sunflower seed oil polyglyceryl-6 ester or mixtures thereof.

11. A cosmetic preparation according to one of the preceding claims, **characterised in that** the preparation contains at least one filler selected from the group consisting of: polymethylsilsesquioxane, dimethicone/vinyldimethicone crosspolymer, dimethicone/bis-isobutyl PPG-20 crosspolymer, dimethicone crosspolymer, mica, silica, nylon-12, PMMA, boron nitride, polyethylene, HDI-trimethylol hexyllactone crosspolymer, magnesium stearate, zinc oxide or mixtures thereof.

12. A cosmetic preparation according to one of the preceding claims, **characterised in that** the preparation can be shaped to form a stick.

13. Use of a cosmetic preparation according to one of claims 1 to 12 as an eyeliner, eyeshadow, eyebrow pencil, tattoo, lip liner, lipstick, mascara, blusher or concealer.

14. Use of a cosmetic preparation according to one of claims 1 to 12 for the production of a cosmetic pencil.

## Revendications

1. Préparation cosmétique contenant au moins une cire de silsesquioxane de formule
(R₂R'SiO_{1/2})ₓ(R"SiO_{3/2})_{y}
dans laquelle R représente à chaque fois un groupe alkyle de 1 à 8 atomes C, R' représente un groupe alkyle monovalent de 9 à 46 atomes C, R" représente un groupe alkyle monovalent de 1 à 8 atomes C ou un groupe aryle, x et y peuvent prendre des valeurs comprises entre 0,05 et 0,95, et au moins un thermoplastique comprenant au moins une unité dérivée de cyclopentadiène, le thermoplastique étant le polydicyclopentadiène hydrogéné.

2. Préparation cosmétique selon la revendication 1, **caractérisée en ce que**, dans la cire de silsesquioxane, R représente à chaque fois un groupe méthyle, R' représente un groupe alkyle de 30 à 46 atomes C, et R" représente un groupe alkyle de 3 atomes C.

3. Préparation cosmétique selon l'une quelconque des revendications précédentes, **caractérisée en ce que** la préparation est anhydre.

4. Préparation cosmétique selon l'une quelconque des revendications précédentes, **caractérisée en ce que** la préparation contient au moins un composant volatil à température ambiante.

5. Préparation cosmétique selon la revendication 4, **caractérisée en ce que** le composant volatil est choisi dans le groupe constitué par : les polydiméthylsiloxanes cycliques, tels que p. ex. le cyclotétrasiloxane, le cyclopentasiloxane, le cyclohexasiloxane, les polydiméthylsiloxanes linéaires, les hydrocarbures volatils de 5 à 16 atomes C, tels que p. eux. : l'isododécane ou l'isohexadécane ; les polydécènes, les polyisobutènes, les polyisobutènes hydrogénés ou les poly(oléfines en C6-14) hydrogénées ou leurs mélanges.

6. Préparation cosmétique selon l'une quelconque des revendications précédentes, **caractérisée en ce que** la préparation contient au moins un hydrocarbure polymère hydrogéné.

7. Préparation cosmétique selon la revendication 6, **caractérisée en ce que** l'hydrocarbure polymère hydrogéné est choisi dans le groupe constitué par : le polyisobutène, le polydécène, les polyoléfines comprenant des unités de 6 à 14 atomes C, le polybutène, le polyéthylène, le polystyrène, le polyisoprène, leurs copolymères ou leurs mélanges.

8. Préparation cosmétique selon l'une quelconque des revendications précédentes, **caractérisée en ce que** la préparation contient au moins un ester de polyglycéryle.

9. Préparation cosmétique selon la revendication 8, **caractérisée en ce que** l'ester de polyglycéryle comprend 3 à 10 unités glycéryle.

10. Préparation cosmétique selon la revendication 8 ou 9, **caractérisée en ce que** l'ester de glycéryle est choisi dans le groupe constitué par : l'ester de polyglycéryle-6 d'huile de noyau d'abricot, l'ester de polyglycéryle-10 d'huile de noyau d'abricot, l'ester de polyglycéryle-4 d'huile de babassu, l'ester de polyglycéryle-6 d'huile de babassu, le bis-butyldiméthicone polyglycéryle-3, l'ester de polyglycéryle-6 d'huile de graines de bourrache, l'ester de polyglycéryle-3 de candelilla/jojoba/son de riz, l'ester de polyglycéryle-6 de beurre de cacao, l'ester de polyglycéryle-6 d'huile de coco, l'ester de polyglycéryle-6 d'huile de graines de café, le dilinoléate dimère de diisostéaroyle polyglycéryle-3, l'ester isostéarate/béhénate de glycéryl/polyglycéryle-6, l'isopolyglycéryl-3-diméthicone, l'isopolyglycéryl-3-diméthiconol, l'éther de lauryle polyglycéryle-6 cétéaryle glycol, l'ester béhénate de polyglycéryle-6 d'huile de graines de macadamia, l'ester de polyglycéryle-4 d'huile d'olive, l'ester de polyglycéryle-6 d'huile d'olive, le polyglycéryle-3 de cire d'abeilles, le béhénate de polyglycéryle-6, le béhénate/eicosadioate de polyglycéryle-10, l'ester d'acide en C₁₂₋₂₀ de polyglycéryle-8, le caprate de polyglycéryle-3, le caprate de polyglycéryle-4, le caprate de polyglycéryle-5, le caprate de polyglycéryle- 6, le caprate de polyglycéryle-10, le caprylate de polyglycéryle-3, le caprylate de polyglycéryle-4, le caprylate de polyglycéryle-6, le caprylate de polyglycéryle-10, le cocoate de polyglycéryle-3, le cocoate de polyglycéryle-4, le décaérucate/décaisostéarate/décaricinoléate de polyglycéryle-8, le décaéthylhexanoate de polyglycéryle-10, le décahydroxystéarate de polyglycéryle-10, le décaisostéarate de polyglycéryle-10, le décastéarate de polyglycéryle-10, le dicaprate de polyglycéryle-3, le diisostéarate de polyglycéryle-3, le diisostéarate de polyglycéryle-6, le diisostéarate de polyglycéryle-10, le dilaurate de polyglycéryle-4, le dilaurate de polyglycéryle-5, le dimyristate de polyglycéryle-10, le dioléate de polyglycéryle-3, le dioléate de polyglycéryle-5, le dioléate de polyglycéryle-6, le dioléate de polyglycéryle-10, le dipalmitate de polyglycéryle-6, le dipalmitate de polyglycéryle-10, la polyglycéryle-3 disiloxane diméthicone, le distéarate de polyglycéryle-3, l'hexaérucate de polyglycéryle-10, l'hexaisostéarate de polyglycéryle-10, l'hexaoléate de polyglycéryle-6, l'hexaoléate de polyglycéryle-10, l'hexastéarate de polyglycéryle-5, l'isostéarate de polyglycéryle-3, l'isostéarate de polyglycéryle-4, l'isostéarate de polyglycéryle-5, l'isostéarate de polyglycéryle-6, l'isostéarate/laurate de polyglycéryle-4, le polymère croisé de polyglycéryle-3/laurylpolydiméthylsiloxyéthyldiméthicone, le linoléate de polyglycéryle-10, le distéarate de polyglycéryle-3 méthylglucose, le myristate de polyglycéryle-3, le myristate de polyglycéryle-5, l'oléate de polyglycéryle-3, l'oléate de polyglycéryle-4, l'oléate de polyglycéryle-10, le palmitate de polyglycéryle-3, le palmitate de polyglycéryle-6, le palmitate de polyglycéryle-10, le pentaisostéarate de polyglycéryle-10, le pentalaurate de polyglycéryle-10, le pentamyristate de polyglycéryle-5, le pentaricinoléate de polyglycéryle-3, le pentaricinoléate de polyglycéryle-6, le pentastéarate de polyglycéryle-4, le pentastéarate de polyglycéryle-6, la polyglycéryle-3 polydiméthylsiloxyéthyldiméthicone, le polyhydroxystéarate de polyglycéryle-6, le diisostéarate /polyhydroxystéarate/sébacate de polyglycéryle-4, le polyricinoléate de polyglycéryle-3, le polyricinoléate de polyglycéryle-4, le sesquiisostéarate de polyglycéryle-6, le sesquistéarate de polyglycéryle-6, le stéarate de polyglycéryle-3, le stéarate de polyglycéryle-4, le tétrabéhénate de polyglycéryle-6, le tétracaprylate de polyglycéryle-6, le tribéhénate de polyglycéryle-5, le triisostéarate de polyglycéryle-3, le trimyristate de polyglycéryle-5, le tristéarate de polyglycéryle-4, le tristéarate de polyglycéryle-5, l'ester de polyglycéryle-6 d'huile de carthame, l'ester de polyglycéryle-6 d'huile de sésame, l'ester de polyglycéryle-6 de beurre de karité, l'ester de polyglycéryle-6 d'huile de haricots de soja, l'ester de polyglycéryle-6 d'huile de graines de tournesol ou leurs mélanges.

11. Préparation cosmétique selon l'une quelconque des revendications précédentes, **caractérisée en ce que** la préparation contient au moins un agent de charge, choisi dans le groupe constitué par : le polyméthylsilsesquioxane, le polymère croisé de diméthicone/vinyldiméthicone, le polymère croisé de diméthicone/bis-isobutyle PPG-20, le polymère croisé de diméthicone, le mica, la silice, le nylon 12, le PMMA, le nitrure de bore, le polyéthylène, le polymère croisé d'HDI/triméthylol hexyllactone, le stéarate de magnésium, l'oxyde de zinc ou leurs mélanges.

12. Préparation cosmétique selon l'une quelconque des revendications précédentes, **caractérisée en ce que** la préparation est façonnable en une mine.

13. Utilisation d'une préparation cosmétique selon l'une quelconque des revendications 1 à 12 en tant que ligneur, ombre à paupières, crayon à sourcils, tatouage, crayon contour des lèvres, rouge à lèvres, mascara, fard à joues ou matériau de couverture.

14. Utilisation d'une préparation cosmétique selon l'une quelconque des revendications 1 à 12 pour la fabrication d'un stylo cosmétique.
